# FASCICULE DE BREVET EUROPEEN

(11) **EP 3 123 989 B1**
(45) Date de publication et mention de la délivrance du brevet: **13.06.2018**
(21) Numéro de dépôt: 16176536.7
(22) Date de dépôt: 28.06.2016
(51) Int. Cl.: A61F 13/06, A61F 13/08, D04B 1/26

(54) **ORTHÈSE DE CONTENTION**
STÜTZORTHESE
RESTRICTIVE ORTHOSIS

(30) Priorité: 30.07.2015 FR 1557342
(43) Date de publication de la demande: 01.02.2017
(73) Titulaire: RICHARD FRERES, 42530 Saint Genest Lerpt (FR)
(72) Inventeur: RICHARD, Dominique, 42110 AUREC/LOIRE (FR); TRAVARD, Philippe, 42153 RIORGES (FR); MARQUAIRE, Laurend, 42100 SAINT ETIENNE (FR)
(74) Mandataire: Delorme, Nicolas

(56) Documents cités:
- EP-A1- 0 498 062
- EP-A1- 2 436 276
- WO-A1-2009/071894
- DE-A1- 10 260 694
- DE-U1-202015 101 990
- GB-A- 2 241 647
- GB-A- 2 473 321

## Description

La présente invention concerne une orthèse de contention.

Il est connu d'utiliser une orthèse de contention pour traiter certaines atteintes articulaires, comme par exemple les luxations.

D'une manière classique, les orthèses de contention se présentent sous la forme d'une gaine élastique configurée pour enserrer une articulation. D'une manière traditionnelle, l'orthèse de contention se présente sous la forme d'une gaine en textile élastique. L'élasticité du textile permet d'enserrer de manière homogène l'articulation. Cependant, dans les zones de plis articulaires comme le pli poplité, les plis des gaines élastiques peuvent provoquer fréquemment des gènes, douleurs ou irritations.

Pour éviter ces désagréments, il est connu d'utiliser des orthèses présentant une ouverture au niveau du pli articulaire. Ces orthèse permettent d'éviter les douleurs et irritations dans les zones de plis articulaires. Cependant, l'ouverture de l'orthèse ne permet pas d'enserrer correctement l'articulation.

En conséquence, la présente invention a pour objectif de proposer une orthèse qui permette d'enserrer correctement l'articulation tout en préservant certaines zones articulaires des douleurs et irritations.

Selon une définition générale, l'invention concerne une orthèse de contention qui comprend une première zone tricotée comprenant un premier fil, un deuxième fil et un fil élastique de trame inséré entre le premier, et le deuxième, fil, et une deuxième zone dans laquelle le premier fil forme une nappe intérieure tricotée, le deuxième fil forme une nappe extérieure tricotée et le fil élastique de trame présente au moins un flotté qui forme une nappe intermédiaire entre la nappe intérieure et la nappe extérieure.

Le tricot de la première zone permet à l'orthèse selon l'invention de garantir une contention optimale de l'articulation. La structure de la deuxième zone permet aux trois nappes de glisser les unes par rapport aux autres ce qui permet d'éviter la formation de plis irritants, tout en garantissant la bonne tenue de l'articulation.

Ainsi, l'invention propose une orthèse qui permet d'enserrer correctement l'articulation tout en préservant certaines zones articulaires des douleurs et irritations.

D'une manière optimale, selon une disposition particulière, la densité de fil élastique de trame de la deuxième zone peut être inférieure à la densité de fil élastique de trame de la première zone.

Cette disposition technique permet de garantir une contention optimale dans la première zone tout en réduisant les risques d'irritation de la peau positionnée contre la deuxième zone.

La nappe intérieure peut présenter un tricot en jersey.

Ainsi la nappe tricotée intérieure peut présenter un aspect doux pour la peau.

Le fil élastique de trame peut présenter une âme fortement élastique et un guipage.

Le premier fil peut être réalisé dans un matériau choisi parmi le groupe comprenant : le coton et les fibres synthétiques.

Le deuxième fil peut être réalisé dans un matériau choisi parmi le groupe comprenant : le coton et les fibres synthétiques.

Selon un mode de réalisation, l'orthèse peut être une genouillère sensiblement tubulaire qui peut présenter au moins un coussin ovale de soutien rotulien diamétralement opposé à la deuxième zone.

Selon le même mode de réalisation, l'orthèse peut comprendre deux baleines latérales diamétralement opposées l'une par rapport à l'autre.

D'autres caractéristiques et avantages de la présente invention se dégageront de la description qui va suivre en regard des dessins annexés qui représentent à titre d'exemple non limitatif une forme de réalisation de l'invention.
- La figure 1 est une vue en perspective d'une orthèse selon l'invention ;
- La figure 2 est une vue de face d'une orthèse selon l'invention ;
- La figure 3 est une vue de derrière d'une orthèse selon l'invention ;
- La figure 4 est une vue de face de l'intérieure d'une orthèse selon l'invention ;
- La figure 5 est une vue de derrière de l'intérieur d'une orthèse selon l'invention dans laquelle la nappe interne de la deuxième zone est déchirée ;
- La figure 6 est une vue détaille d'une deuxième zone d'une orthèse selon l'invention dans laquelle la nappe interne est déchirée pour laisser voir la nappe intermédiaire et la nappe externe ;
- La figure 7 est une vue schématique en coupe de la première zone tricotée et la deuxième zone d'une orthèse selon l'invention ;
- La figure 8 est une vue schématique en coupe de la nappe intermédiaire d'une orthèse selon l'invention.

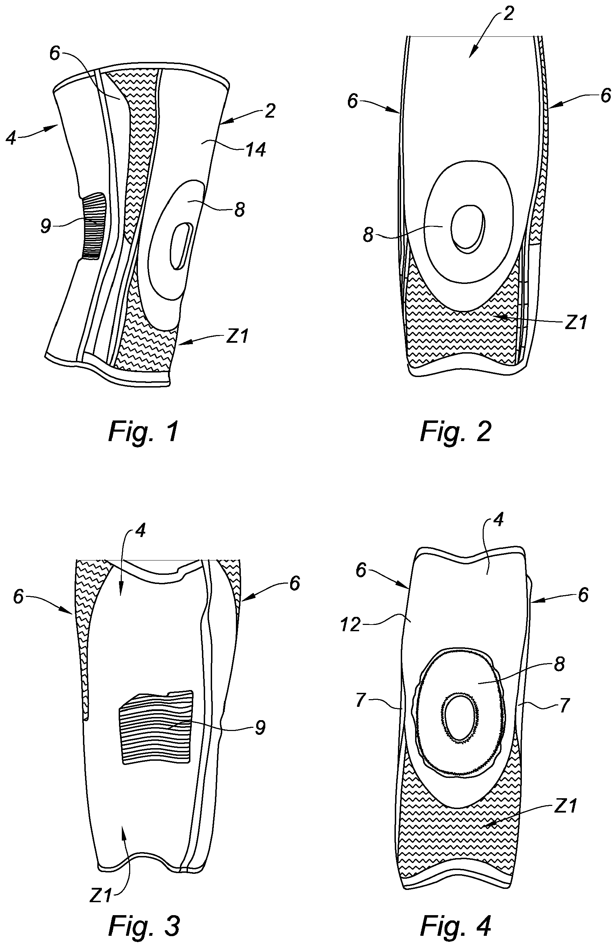

En référence aux figures 1 à 3, l'invention concerne une orthèse 1 de contention. Selon le mode de réalisation ici présenté, l'orthèse 1 est une genouillère. Cependant, selon d'autres modes de réalisation, l'orthèse 1 peut par exemple être une chevillière, une coudière ou une épaulière.

Comme on peut l'observer sur les figures 1 à 3, l'orthèse 1 présente une géométrie sensiblement tubulaire avec, une portion antérieure 2, une portion postérieure 4 et deux portions latérales 6.

La portion antérieure 2 peut présenter un coussin ovale de soutien rotulien 8 représenté sur les figures 1 et 4.

La portion postérieure 4 présente une surface sensiblement rectangulaire 9 qui sera détaillée ultérieurement. Cette surface est diamétralement opposée au coussin ovale de soutien rotulien 8.

Les portions latérales peuvent 6 chacune présenter une baleine 7.

Comme on peut le voir sur les figures 1 à 6, l'orthèse 1 présente une première zone Z1 tricotée.

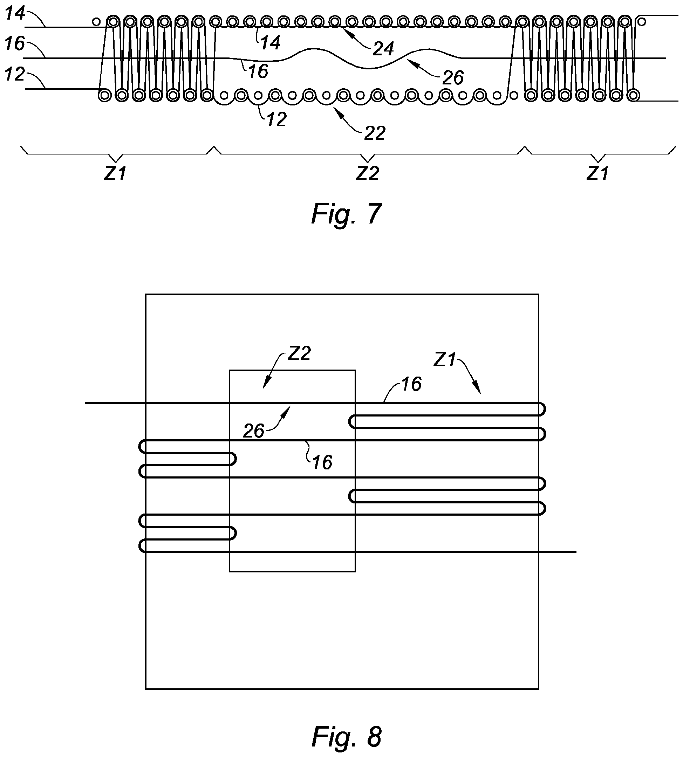

On peut se reporter à la figure 7 pour apprécier le tricotage de la première zone Z1. La première zone Z1 tricotée comprend un premier fil 12, un deuxième fil 14 et un fil élastique de trame 16 inséré entre le premier, et le deuxième, fil 12-14.

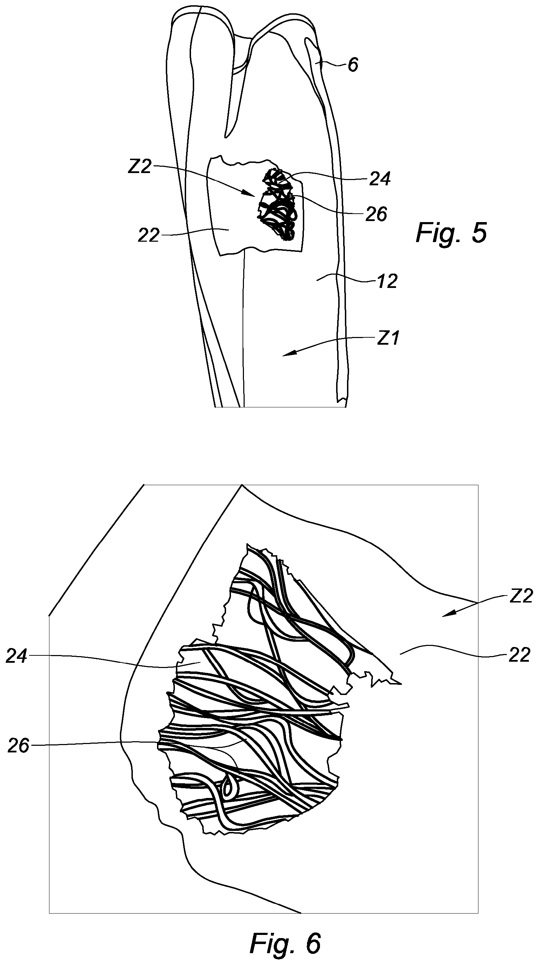

Dans l'exemple ici présenté, le premier fil 12 définit une surface intérieure de l'orthèse 1, visible sur les figures 4 et 5. Le deuxième fil 14 définit une surface extérieure de l'orthèse 1. Le fil élastique de trame 16 permet à l'orthèse 1 d'avoir des propriétés élastiques de contention.

Selon un mode de réalisation, les premier et deuxième fils 12-14 sont en polyamide. Cependant, selon d'autres modes de réalisation, les premiers et deuxième fils peuvent être, par exemple, en polyester ou en fibres synthétiques. De plus, le fil élastique de trame 16 peut présenter une âme en gomme fortement élastique et un guipage en coton ou en polyamide.

De plus, comme on peut le voir plus particulièrement sur les figures 5 à 7, la surface sensiblement rectangulaire 9 correspond à une deuxième zone Z2 dans laquelle le premier fil 12 forme une nappe tricotée intérieure 22, le deuxième fil 14 forme une nappe tricotée extérieure 24 et le fil élastique de trame 16 présente au moins un flotté qui forme une nappe intermédiaire 26 entre la nappe tricotée intérieure 22 et la nappe tricotée extérieure 24.

Comme on peut le remarquer sur la figure 8, la densité de fil élastique de trame 16 de la deuxième zone Z2 est inférieure à la densité de fil élastique de trame 16 de la première zone Z1. Il s'agit là d'une disposition technique particulièrement avantageuse de l'invention. En effet, la densité réduite de fil élastique de trame 16 dans la nappe intermédiaire 26 et la liberté de la nappe intermédiaire 26 par rapport aux nappes tricotées intérieure 22 et extérieure 24, permet de préserver la peau qui est en contact avec la deuxième zone Z2 d'éventuels irritations.

Dans l'exemple ici présenté, la densité de fil élastique de trame 16 dans la deuxième zone Z2 est deux fois moindre à la densité de fil élastique de trame 16 dans la première zone Z1.

En outre, selon le mode de réalisation ici présenté, la nappe intérieure 22 présente un tricot en jersey. Ainsi la nappe intérieure 22 présente un aspect doux pour la peau.

A l'usage, l'orthèse 1 peut être positionnée autour d'un genou.

Le coussin ovale de soutien rotulien 8 est positionné contre la rotule et la deuxième zone Z2 est positionnée contre le pli poplité du genou.

Le tricot de la première zone Z1 permet à l'orthèse 1 de garantir une contention optimale du genou. De plus, dans la deuxième zone Z2, les nappes intérieure 22, intermédiaire 26 et extérieure 24, peuvent glisser les unes par rapport aux autres, ce qui évite la formation de plis irritants, tout en garantissant la bonne tenue du genou.

Ainsi, l'invention propose une orthèse qui permet d'enserrer correctement l'articulation tout en préservant certaines zones articulaires des douleurs et irritations.

Bien entendu, l'invention ne se limite pas à la seule forme d'exécution représentée ci-dessus, mais elle embrasse au contraire toutes les formes de réalisation.

## Revendications

1. Orthèse (1) de contention **caractérisée en ce que** l'orthèse de contention (1) comprend une première zone (Z1) tricotée comprenant un premier fil (12), un deuxième fil (14) et un fil élastique de trame (16) inséré entre les premier (12) et deuxième (14) fils, et une deuxième zone (Z2) dans laquelle le premier fil (12) forme une nappe intérieure (22) tricotée, le deuxième fil (14) forme une nappe extérieure (24) tricotée et le fil élastique de trame (16) présente au moins un flotté qui forme une nappe intermédiaire (26) entre la nappe intérieure (22) et la nappe extérieure (24).

2. Orthèse (1) de contention selon la revendication 1, **caractérisée en ce que** la densité de fil élastique de trame (16) de la deuxième zone (Z2) est inférieure à la densité de fil élastique de trame (16) de la première zone (Z1).

3. Orthèse (1) de contention selon l'une des revendications 1 ou 2, **caractérisée en ce que** la nappe intérieure (22) présente un tricot en jersey.

4. Orthèse (1) de contention selon l'une des revendications 1 à 3, **caractérisée en ce que** le fil élastique de trame (16) présente une âme fortement élastique et un guipage.

5. Orthèse (1) de contention selon l'une des revendications 1 à 4, **caractérisée en ce que** le premier fil (12) est réalisé dans un matériau choisi parmi le groupe comprenant : le coton et les fibres synthétiques.

6. Orthèse (1) de contention selon l'une des revendications 1 à 5, **caractérisé en ce que** le deuxième fil (14) est réalisé dans un matériau choisi parmi le groupe comprenant : le coton et les fibres synthétiques.

7. Orthèse (1) de contention selon l'une des revendications 1 à 6, **caractérisée en ce que** l'orthèse (1) est une genouillère sensiblement tubulaire qui présente au moins un coussin ovale de soutien rotulien (9) diamétralement opposé à la deuxième zone (Z2).

8. Orthèse (1) selon la revendication 7, **caractérisée en ce qu'**elle comprend deux baleines latérales (7) diamétralement opposées l'une par rapport à l'autre.

## Patentansprüche

1. Stützorthese (1), **dadurch gekennzeichnet, dass** die Stützorthese (1) eine erste gestrickte Zone (Z1) umfasst, umfassend einen ersten Faden (12), einen zweiten Faden (14) und einen elastischen Schussfaden (16), der zwischen den ersten (12) und zweiten (14) Faden eingeführt ist, und eine zweite Zone (Z2), in der der erste Faden (12) eine gestrickte innere Lage (22) bildet, der zweite Faden (14) eine gestrickte äußere Lage (24) bildet und der elastische Schussfaden (16) mindestens eine Flotte darstellt, die eine Zwischenlage (26) zwischen der inneren Lage (22) und der äußeren Lage (24) bildet.

2. Stützorthese (1) nach Anspruch 1, **dadurch gekennzeichnet, dass** die Dichte des elastischen Schussfadens (16) der zweiten Zone (Z2) geringer als die Dichte des elastischen Schussfadens (16) der ersten Zone (Z1) ist.

3. Stützorthese (1) nach Anspruch 1 oder 2, **dadurch gekennzeichnet, dass** die innere Lage (22) eine Jersey-Strickware darstellt.

4. Stützorthese (1) nach einem der Ansprüche 1 bis 3, **dadurch gekennzeichnet, dass** der elastische Schussfaden (16) einen stark elastischen Kern und eine Umspinnung darstellt.

5. Stützorthese (1) nach einem der Ansprüche 1 bis 4, **dadurch gekennzeichnet, dass** der erste Faden (12) aus einem Material hergestellt ist, ausgewählt aus der Gruppe, umfassend: Baumwolle und synthetische Fasern.

6. Stützorthese (1) nach einem der Ansprüche 1 bis 5, **dadurch gekennzeichnet, dass** der zweite Faden (14) aus einem Material hergestellt ist, ausgewählt aus der Gruppe, umfassend: Baumwolle und synthetische Fasern.

7. Stützorthese (1) nach einem der Ansprüche 1 bis 6, **dadurch gekennzeichnet, dass** die Orthese (1) ein im Wesentlichen rohrförmiger Knieschutz ist, der mindestens ein ovales Kniescheiben-Stützkissen (9) darstellt, das der zweiten Zone (Z2) diametral gegenüber liegt.

8. Orthese (1) nach Anspruch 7, **dadurch gekennzeichnet, dass** sie zwei seitliche Formbügel (7) umfasst, die einander diametral gegenüber liegen.

## Claims

1. A compressive orthosis (1) **characterized in that** the compressive orthosis (1) comprises a first knitted area (Z1) comprising a first yarn (12), a second yarn (14) and a weft elastic yarn (16) inserted between the first (12) and second (14) yarns, and a second area (Z2) in which the first yarn (12) forms an inner knitted layer (22), the second yarn (14) forms an outer knitted layer (24) and the elastic weft yarn (16) has at least one float that forms an intermediate layer (26) between the inner layer (22) and the outer layer (24).

2. The compressive orthosis (1) according to claim 1, **characterized in that** that the elastic weft yarn (16) density of the second area (Z2) is less than the elastic weft yarn (16) density of the first area (Z1).

3. The compressive orthosis (1) according to any of claims 1 or 2, **characterized in that** the inner layer (22) has a jersey knit.

4. The compressive orthosis (1) according to any of claims 1 to 3, **characterized in that** the elastic weft yarn (16) has a highly elastic core and a covering.

5. The compressive orthosis (1) according to any of claims 1 to 4, **characterized in that** the first yarn (12) is made of a material selected from the group comprising: cotton and synthetic fibers.

6. The compressive orthosis (1) according to any of claims 1 to 5, **characterized in that** the second yarn (14) is made of a material selected from group comprising: cotton and synthetic fibers.

7. The compressive orthosis (1) according to any of claims 1 to 6, **characterized in that** the orthosis (1) is a substantially tubular knee brace which has at least one oval patellar support cushion (9) diametrically opposite to the second area (Z2).

8. The compressive orthosis (1) according to claim 7, **characterized in that** it comprises two lateral stays (7) diametrically opposite to each other.
